Europäisches Patentamt

**European Patent Office**

**Office européen des brevets**

(19)

(11) Publication number: **0 204 977**

**A1**

(12) # EUROPEAN PATENT APPLICATION

(21) Application number: **86106427.7**

(22) Date of filing: **12.05.86**

(51) Int. Cl.⁴: **A 61 M 5/14**

(30) Priority: **14.05.85 US 734028**

(43) Date of publication of application:
**17.12.86 Bulletin 86/51**

(84) Designated Contracting States:
**AT BE CH DE FR GB IT LI LU NL SE**

(71) Applicant: **INTELLIGENT MEDICINE, INC.**
**253 Adams Street**
**Denver, Colorado 80206(US)**

(72) Inventor: **Howson, David C.**
**254 Adams St.**
**Denver Colorado(US)**

(72) Inventor: **Fellinger, Michael W.**
**1590 Quince Avenue**
**Boulder Colorado(US)**

(72) Inventor: **Popken, John A.**
**912 Corey St.**
**Longmont Colorado(US)**

(72) Inventor: **Altobellis, Richard M.**
**P.O. Box 954**
**Lyons Colorado(US)**

(74) Representative: **Baillie, Iain Cameron et al,**
**c/o Ladas & Parry Isartorplatz 5**
**D-8000 München 2(DE)**

(54) Syringe drive apparatus and method.

(57) Drive apparatus and method are disclosed for controlling fluid flow from a syringe. A disposable unit that includes a syringe (37) and rack (30) mounted on a retaining clip (47) is positionable on a drive unit so that, when so positioned, the piston of the syringe is precisely displaced to deliver medicament from the syringe (37) to a patient. A non-disposable unit is also disclosed onto which a syringe (47) and piston (48) can be positioned so that the device thereafter also provides precise medicament delivery. The longitudinal movement of the rack (80) is effected by rotating the pinion gear (76) that is connected with a pulse-driven, power efficient stepper motor (82), with the operation of the stepper motor (82) being controlled by a pre-programmed memory unit (126). A hinged rack (160) is also disclosed, as is a one-way ball drive unit (182).

Fig_3

Croydon Printing Company Ltd

SYRINGE DRIVE APPARATUS AND METHOD

Field of the Invention

This invention relates to a drive apparatus and method, and, more particularly, relates to a syringe drive device and method.

Background of the Invention

The use of syringes to retain a fluid, such as a medicament, is well known. Devices and methods have also been heretofore suggested and/or utilized to control delivery of medicament from a syringe by controlling the movement of the syringe piston.

Prior devices and methods for controlling movement of the syringe piston have, however, not proved to be fully effective, at least for all intended purposes. Such prior known devices and/or methods have, for example, been ineffective in precisely controlling medicament delivery, have lacked the ability to deliver changing amounts of medicament over different time periods, have lacked safeguards against undesired use, have not been fully dependable in operation, and/or have lacked simplicity of structure and/or operation.

-2-

## Summary of the Invention

This invention provides an improved device and method for controlling delivery of fluid from a syringe. In the preferred embodiment, precise delivery of fluid from the syringe is effected using a disposable unit insertable into a drive unit, with the disposable unit including both the syringe and a rack. A non-disposable unit is also shown for effecting precise delivery of fluid from a syringe and piston unit insertable into a drive unit having a rack and pinion provided therein.

It is therefore an object of this invention to provide an improved device and method for controlling delivery of fluid from a syringe.

It is another object of this invention to provide an improved device and method for controlling delivery of fluid from a syringe using a rack and pinion.

It is still another object of this invention to provide an improved device and method for controlling delivery of fluid from a syringe utilizing a disposable unit.

It is still another object of this invention to provide an improved device and method for controlling delivery of fluid from a syringe utilizing a disposable unit with a syringe and rack retained by a retaining clip.

-3-

It is still another object of this invention to provide an improved device and method for controlling delivery of fluid from a syringe utilizing a stepper motor for reasons of safety with the stepper motor being driven in a novel power efficient manner.

It is still another object of this invention to provide an improved device and method for controlling delivery of fluid from a syringe utilizing a non-disposable unit that includes a rack and pinion.

With these and other objects in view, which will become apparent to one skilled in the art as the description proceeds, this invention resides in the novel construction, combination, arrangement of parts and method substantially as hereinafter described and more particularly defined by the appended claims, it being understood that changes are meant to be included as come within the scope of the claims.

## Description of the Drawings

The accompanying drawings illustrate complete embodiments of the invention according to the best mode so far devised for the practical application of the principles thereof, and in which:

FIGURE 1 is a perspective view of the syringe drive device of this invention shown with the protective casing in the closed position;

FIGURE 2 is a partial front perspective view of the disposable syringe drive device of this invention shown with the protective casing in the open position and illustrating the disposable syringe assembly operatively placed within the casing;

FIGURE 3 is a rear perspective view of the syringe drive device shown in FIGURE 2 with the lid cut away for illustrative purposes;

FIGURE 4 is a top perspective view of the syringe drive device as shown in FIGURE 3 except that the disposable syringe assembly has been removed from the device;

FIGURE 5 is a partial perspective view of the disposable syringe assembly shown in FIGURES 2 and 3 and illustrating the retaining clip locking feature;

FIGURE 6 is a top view of the syringe drive device as shown in FIGURES 2 through 4 with various structures partially broken away to illustrate gearing for the rack and pinion drive assembly;

FIGURE 7 is a top view of the syringe drive device with the lid removed and four syringes operatively positioned to illustrate different driven stages of the syringes;

FIGURE 8 is a perspective view of the non-disposable syringe drive device of this invention shown with the protective cover in an open position and without syringes operatively positioned within the casing;

FIGURE 9 is an exploded partial side view of the rack and connecting assembly for use with the non-disposable syringe device as shown in FIGURE 8;

FIGURE 10 is a partial side view of the rack and connecting assembly for use with the non-disposable syringe device as shown in FIGURE 8 with the rack shown connected with the syringe piston;

FIGURE 10(A) is a partial perspective view illustrating the rear section of the rack assembly;

FIGURE 11 is a side view of the rack and connecting assembly as shown in FIGURE 10 with the rack shown in position for releasing the piston;

FIGURE 12 is a layout schematic of the syringe drive unit;

FIGURE 13 is a layout schematic illustrating syringe drive and control;

FIGURE 14 is a perspective view of an alternate embodiment of the syringe device using a hinged rack assembly;

FIGURE 15 is an end view of another embodiment of the syringe drive device utilizing a one-way roller drive; and .

FIGURE 16 is a series of timing pulses utilized to drive the stepper motor.

## Description of the Invention

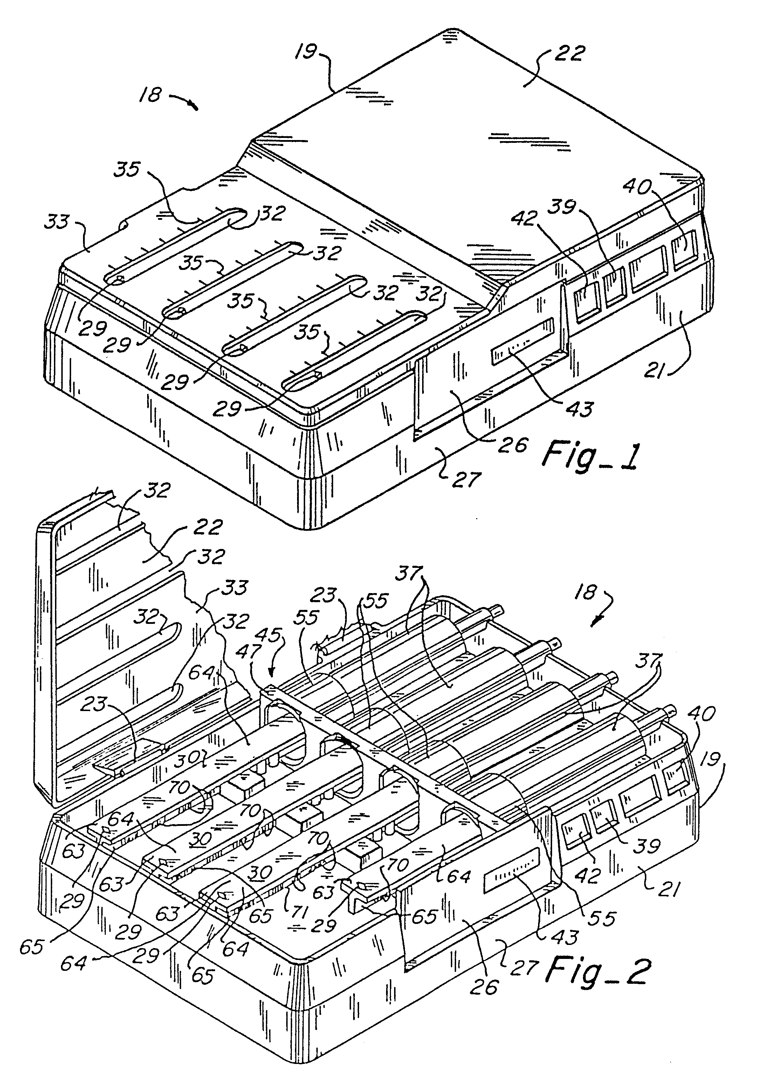

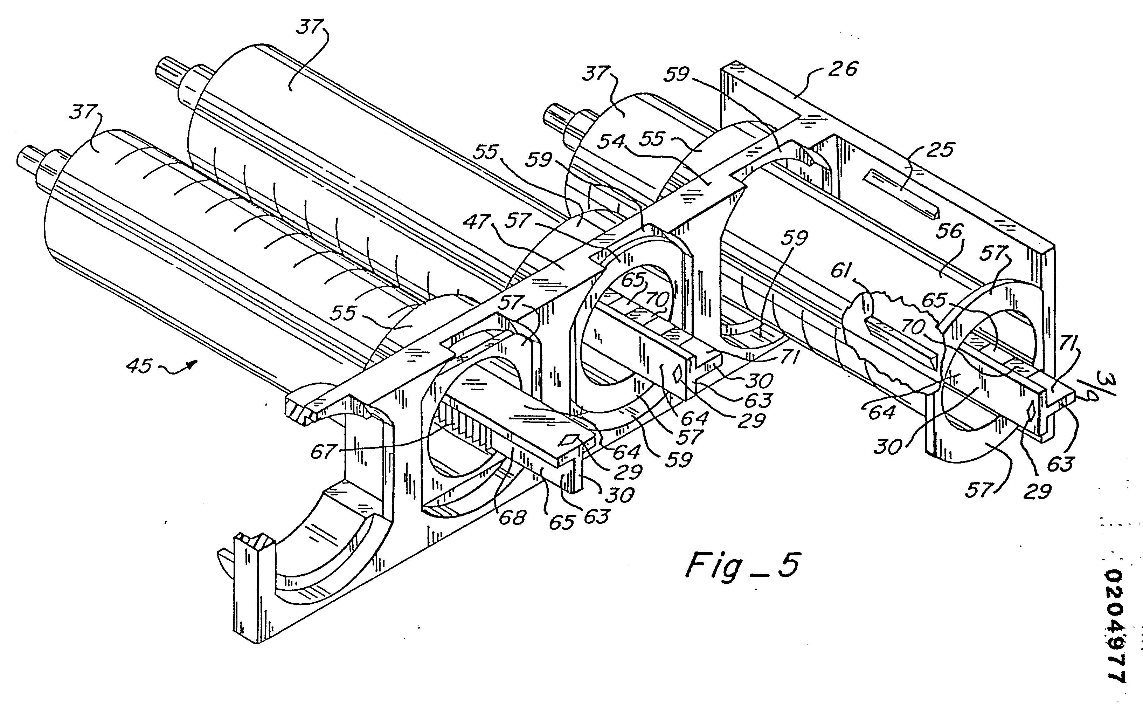

Referring now to the drawings, syringe drive 18 of this invention is shown in FIGURE 1 within casing, or housing, 19. Casing 19, as best shown in FIGURES 1 through 4, includes a base, or bottom, portion 21 and a lid, or top, portion 22 that is hinged by hinges 23 at the rear side 24 of base portion 21. In addition, a retaining, or locking, mechanism 25 (see FIGURE 5) is provided on mounting bracket 26 at the front side 27 of the base portion.

As best shown in FIGURES 1 through 3, a tab, or indicator, 29 is provided on rack 30 so as to be visible through the slotted openings 32 in the top 33 of lid 22. As shown in FIGURE 1, indicia 35 are also preferably utilized at the side of each slotted opening 32 to indicate the amount of use of fluid dispensed from syringe 37 then being driven by rack 30.

As also indicated in FIGURES 1, 2, and 12, depressable start and stop buttons 39 and 40, respectively, are provided at front portion 27 of base portion 21 of the casing 19. In addition, a light emitting diode (LED) enable flasher indicator 42 is provided at base portion 21, and an information plate 43 is also provided at mounting bracket 26.

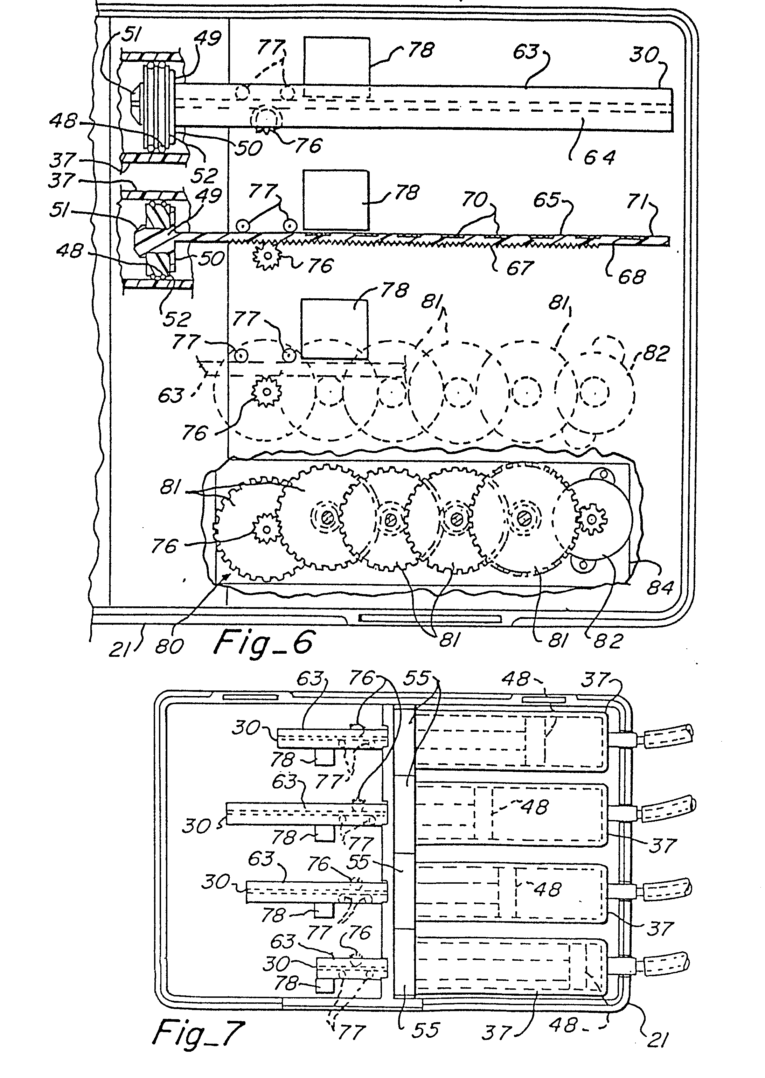

The now preferred embodiment of this invention is shown in FIGURES 2 through 7, and includes a disposable unit 45. Unit 45 includes syringe 37 and rack 30 both of which are mounted on retaining clip 47. Retaining clip 47 preferably receives and retains a plurality of syringes (four are indicated by way of example in FIGURES 1 through 7), and each syringe has a separate rack 30 connected with syringe piston, or plunger, 48 by means of connector 49 which receives centrally apertured piston 48 between disc 50 and end retainer 51 (see FIGURE 6). Piston 48 has sealing lips 32 for engaging the inner surface of the syringe barrel, as is conventional.

As best shown in FIGURE 5, retaining clip 47 includes a mounting wall 54 having a plurality of barrels 55 formed therein to receive the rear end portion 56 of each syringe. As also shown in FIGURE 5, the rear end portion 56 of end syringe has arcuate lip portions 57 at opposite sides which are received in matching portions 59 of each clip barrel 55. After insertion and upon rotation (preferably about $90^{\circ}$ of

the syringe about its central axis), lip portion 57 rotates behind the end of the barrel, and is thereafter locked against movement with respect to the clip until the syringe is further rotated in either direction, so that the lips are again aligned with the matching arcuate portions of the barrel.

Mounting bracket 26 is attached to wall 54 and extends at one side of and parallel to syringes 37. In addition to snap fastener 35 at the top of mounting bracket 26 (for retaining the lid in the closed position), bracket 26 also has a snap fastener 61 protruding inwardly toward the syringes to enable the mounting bracket to be snapped into the base portion of the casing. In addition, bracket 26 preferably has a tamper-proof prescription label base 43 for receiving, as the information displayed, an indication of the prescription for medicaments to be included in the syringes retained by the clip.

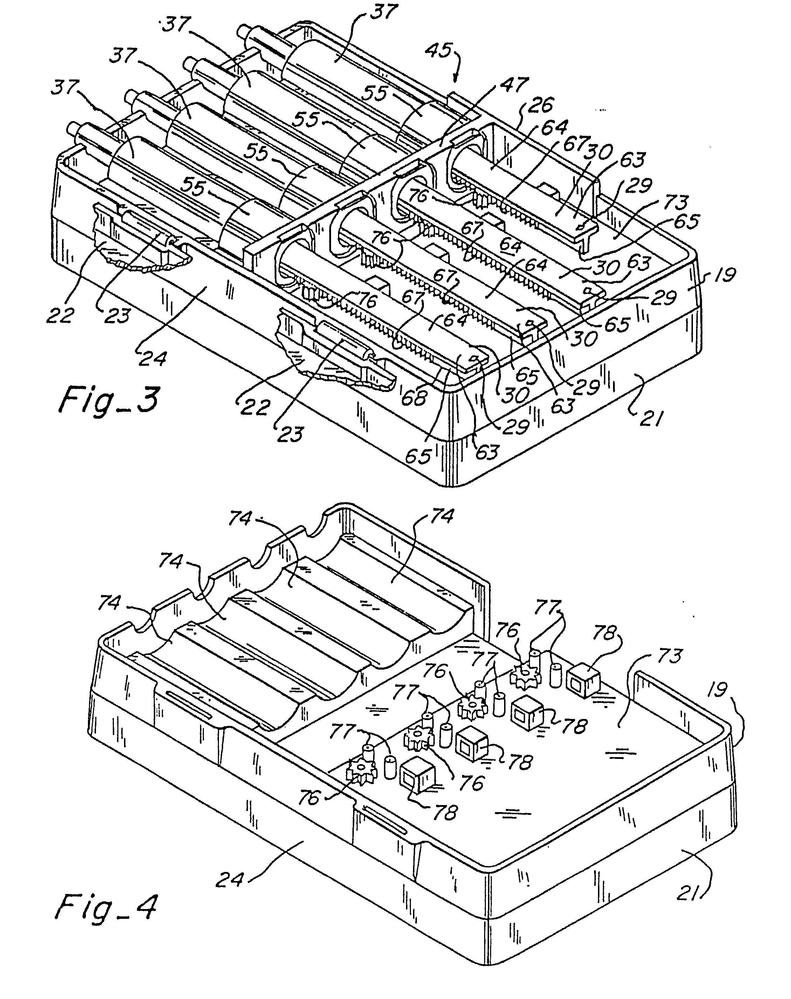

Rack 30 includes an elongated T-shaped beam, or bar, 63, the upper, horizontally extending portion 64 of which has indicator 29 at the rear edge thereof, and the lower, vertically extending portion 65 of which has teeth 67 therein at one side 68. As can be appreciated from FIGURES 3,5 and 6, teeth 67 are relatively small and fine to better control the longitudinal movement of the beam. As shown in FIGURES 2 and 6, a series of distinguishable strips 70 are provided at side 71 of vertical portion 65 of beam 63 to sense beam positioning, as brought out more fully hereinafter.

As best shown in FIGURES 2 through 4, disposable unit 45 is inserted onto base 21 at the top 73 thereof so that the syringes 37 are received in indentions 74 as mounting bracket 26 is snapped into place centrally of the base at the front thereof.

When disposable unit 45 is snapped into position, teeth 67 of each rack 30 engage pinion, or driving, gear 76, which gear extends above top 73 of the base portion of the casing. As also shown in FIGURES 4 and 6, vertical portion 65 of beam 63 extends between driving gear 76 and a pair of guide pins 77 to constrain movement of beam 63 in opposite longitudinal directions when being driven by pinion gear 76. As also shown in FIGURES 4 and 6, optical sensor 78 is positioned adjacent to guide pins 77 (and thus adjacent to side 71 of vertical portion 65 of beam 63) to sense the positioning of the optically distinguishable strips 70 on side 71.

Each rack 30 is independently driven by a separate driving unit 80, as best shown in FIGURE 6. As shown, a series of gears 81 are positioned between pinion gear 76 and stepper motor 82 (as indicated in FIGURES 6 and 13) for enabling precise movement of the rack mechanism 30. As also indicated in FIGURE 6, each driving unit 80 is mounted in a separate gear box 84, which gear box is positioned within base 21 of the casing below top 73.

FIGURE 7 illustrates that the pistons 48 of the plurality of syringes 37 are independently driven through separate gearing 81 and separate pinion gear 76. As shown, each gear box 84 is mounted below and in line with the path of travel of beam 63 as beam 63 drives the associated piston 48 from a retracted position (at the rear of the syringe) to an extended position (at the front of the syringe) during which movement the fluid in the syringe is expelled, or discharged, from the syringe.

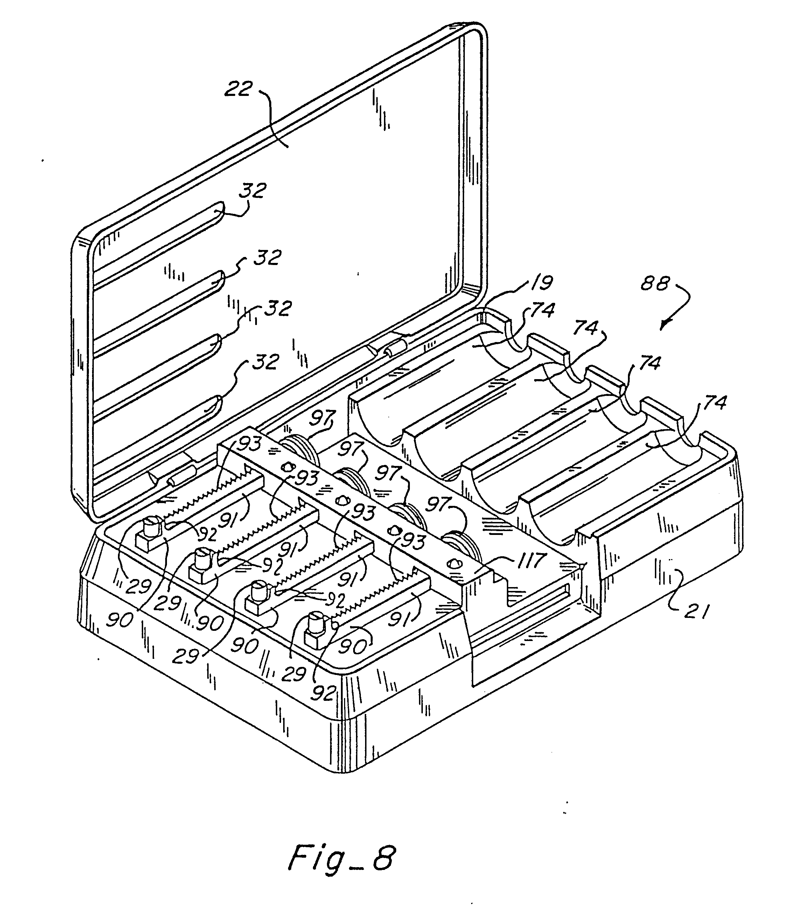

A non-disposable embodiment 88 of the syringe drive device of this invention is shown in FIGURES 8 through 11. As indicated in FIGURE 8, non-disposable embodiment 88 is similar to disposable embodiment 18 (as shown in FIGURES 2 through 7), except that rack 90 is mounted within the casing.

As indicated in FIGURE 8, rack 90 includes indicator 29 which is visible through the slots 32 when the lid 22 of casing 19 is in the closed position (as shown in FIGURE 1).

Rack 90 includes a beam, or bar, 91 having an upstanding shoulder 92 at the rear end portion (which shoulder has indicator 29 mounted at the top thereof). As indicated in FIGURE 8, beam 90 has teeth 93 on one side wall.

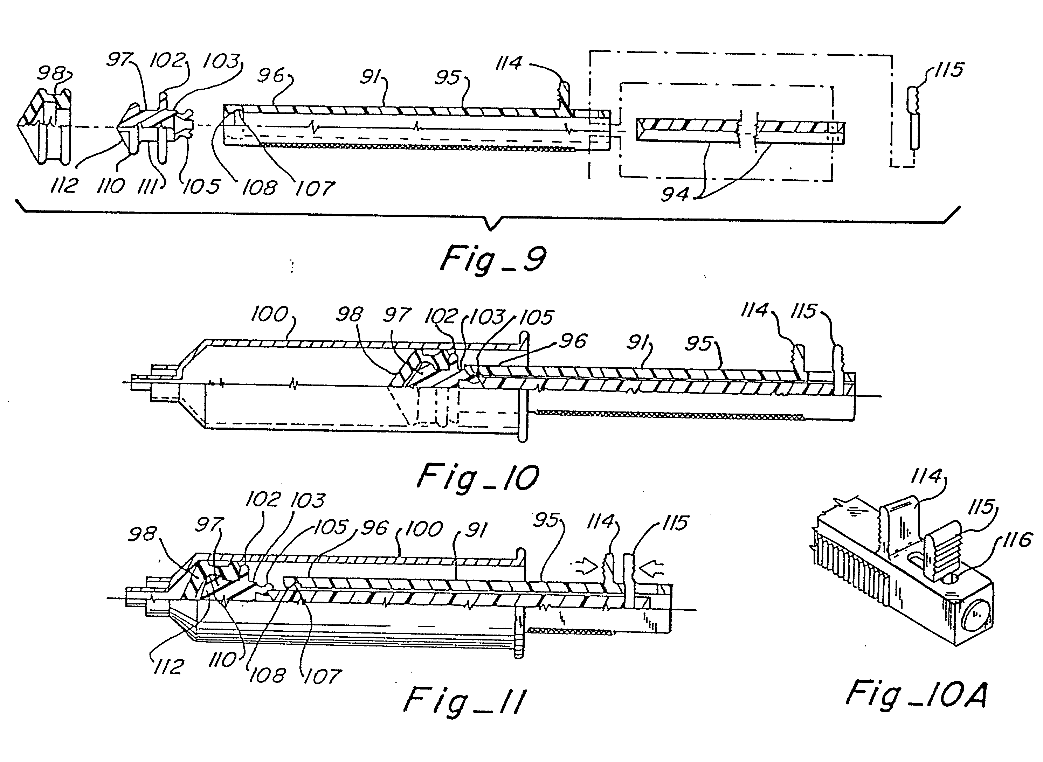

As shown in FIGURE 9, beam 91 preferably includes a central strip 94, which strip is within outer portion 95 of the beam. The forward end 96 of outer portion 95 of beam 91 is adapted to receive tip 97, which tip engages piston 98 of syringe 100.

Tip 97 has a disc 102 thereon that has a rearwardly facing annular connector 103 extending rearwardly therefrom. Connector 103 has a lip 105 thereon, so that as connector 103 is inserted into annular groove 107 (which groove has an annular shoulder 108) at the forward end 96 of beam 91, connector 103 is snapped into the annular groove, as indicated in FIGURE 10. Alternately, the annular connector could have an inwardly directed lip which snaps over an outwardly directed shoulder on end 96 of beam 91.

A smaller disc 110 (i.e., smaller relative to disc 102) is parallel to and spaced forwardly of disc 102 by spacer 111 and terminates in guide 112 which extends forwardly of disc 110 for engaging piston 98. Disc 110 and guide 112 are inserted into piston 98 of the syringe/piston unit, as shown

-12-

in FIGURE 10 when the syringe is operatively positioned on the non-disposable unit. In this embodiment, only the syringe/piston unit is inserted into and removed from the syringe/piston unit.

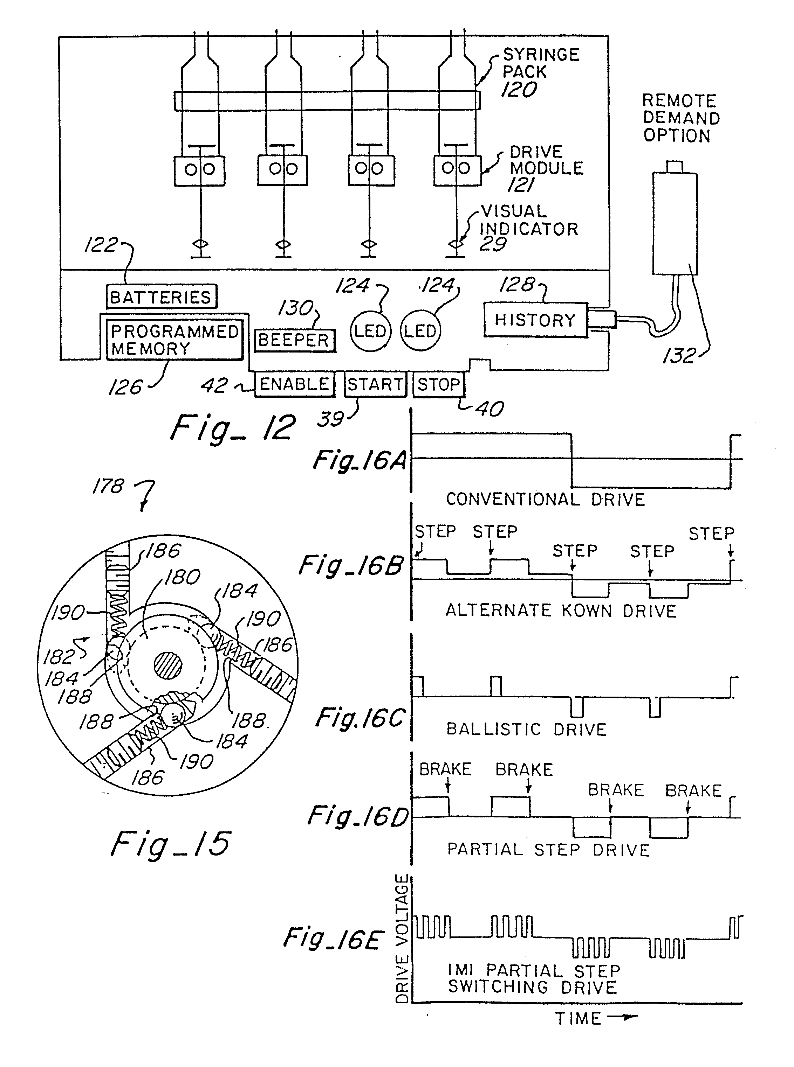

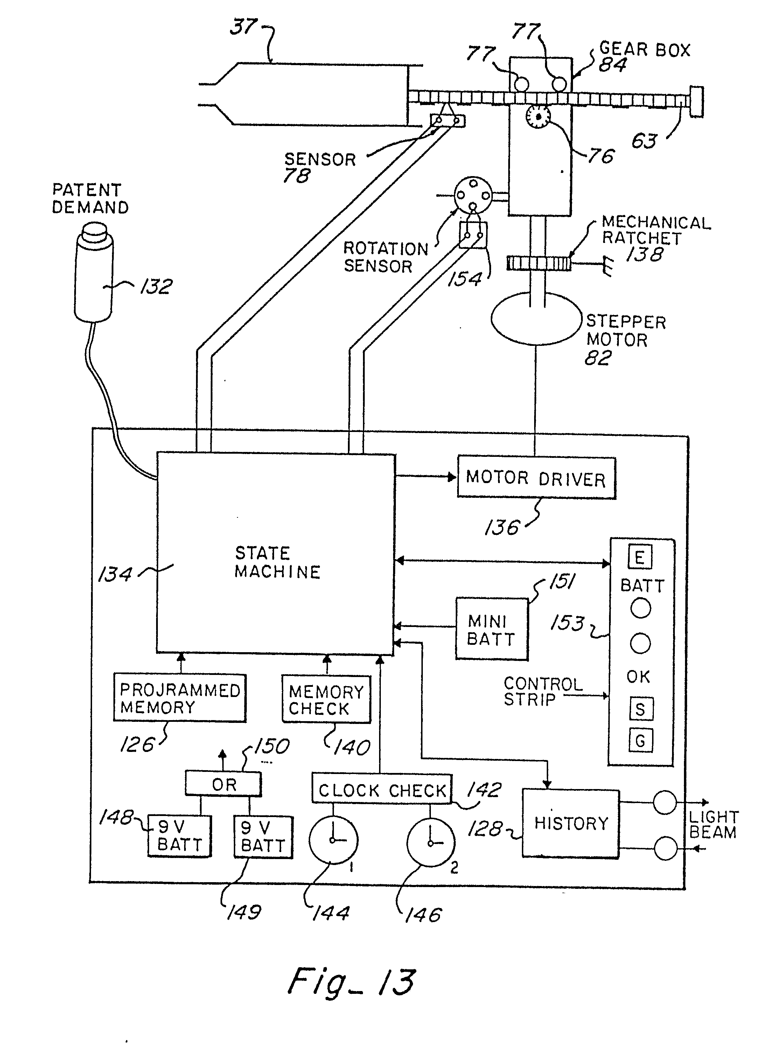

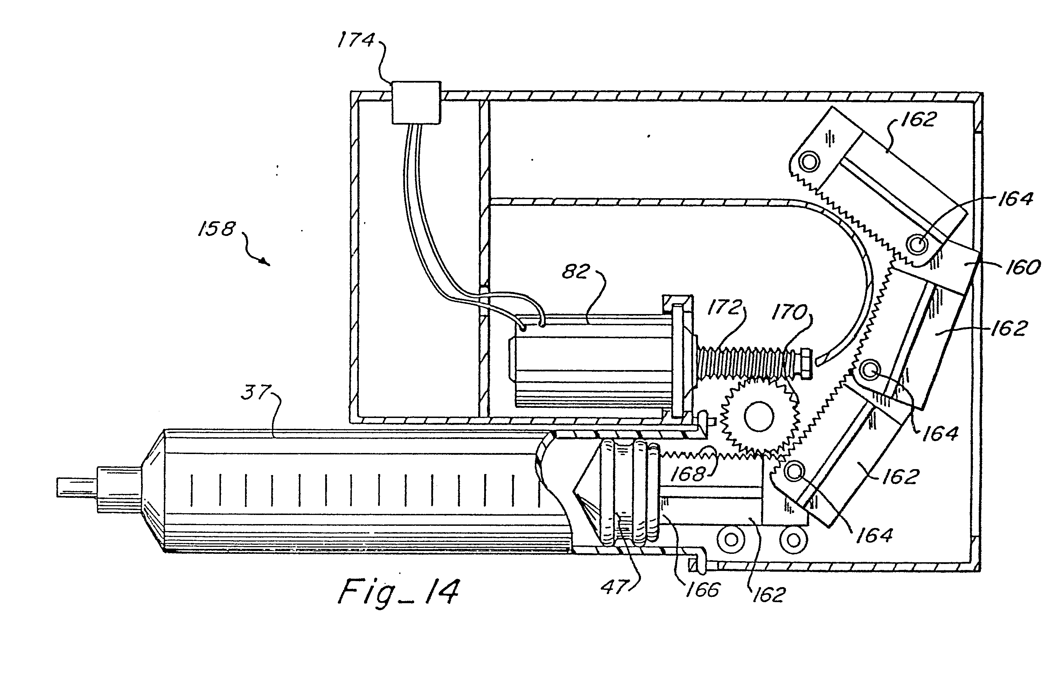

As indicated in FIGURES 10 and 11, outer portion 95 of beam 91 can be retracted with respect to inner portion 94 to enable release of the rack from a syringe/piston unit. As shown best in FIGURE 10(A), a gripping ear 114 is provided on outer portion 95 and gripping ear 115 extends extends through slot 116 so that ear 115 is connected with inner portion 94 of the beam. As the ears are moved toward one another, as indicated in FIGURE 11, this causes the outer portion 95 to be retracted and pulls outer end 96 out of engagement with connector 103 of tip 97.

The driving mechanism for embodiment 88 is the same as shown in connection with the disposable unit of FIGURES 2 through 7 (i.e., pinion gear 76 meshes with teeth 96 of beam 91 to control longitudinal movement of the beam from the piston retracted position to the piston extended position, which movement of the piston effects medicament delivery from the syringe).

As indicated in FIGURE 8, a cover 117 is provided for covering the pinion gears 76 and guide pins 77. While not specifically shown in FIGURE 8, beam 91 preferably also has

-13-

distinguishable strips 70 thereon with strip sensor 78 also being within cover 117.

A layout schematic of the syringe drive device (non-disposable or disposable) is shown in FIGURE 12. As shown, a syringe pack 120 is provided (although each syringe may, if desired, be individually provided), with each syringe containing the predetermined amount of the desired medicament to be dispensed. Pack 120 (or the individual syringes) is placed in the dispensing rack (i.e., the syringes are received in indentations 74 in the top of the base portion of the drive unit) so that the drive modules 121 (i.e., the pinion gears and associated gearing to the stepper motor) drive the piston of each syringe to deliver medicament as needed. The indicators visible at the top of the unit give a visual indication of the amount of medicament delivered at any time from any syringe.

As also indicated in FIGURE 12, the device is battery powered, by means of batteries 122, and preferably includes indicators (LEDs) 124 to indicate satisfactory battery charge.

As also indicated, the syringe drive unit of this invention is controlled by pre-programmed memory unit 126, which unit is preferably a programmable non-volatile logic device, such as a PROM device. It is necessary that unit

-14-

126 be inserted into the device for operation of the device. The pre-programmed memory unit is externally programmed to establish the time and amount of medicament to be delivered by each syringe and controls the operation of the drive unit so that the exact amount is independently delivered from each syringe at the exact required time.

In addition, a history 128 of medicament delivery is made and stored for use in prescribing further medicament delivery, a beeper 130 is provided for alerting purposes, and remote demand option 132 can also be provided to allow a patient to control medicament delivery when, and to the extent, allowed by the pre-programmed memory unit 126.

As indicated by the schematic of FIGURE 13, a state machine 134 controls operation of stepper motor 82 through motor drive 136.

A state machine is a logic network whose output state is determined by the present state of its input and the state of data currently being read from memory. A combinational logic could be utilized in lieu of a state machine. While a microprocessor could also be utilized in lieu of a state machine, it is not preferred due to safety factors.

While only one stepper motor is illustrated in FIGURE 13, it is to be realized that a separate stepper motor is

-15-

provided for each rack 30 utilized.  State machine 134 provides a pulse output through motor driver 136 to each stepper motor so that the drive is thus accomplished as a pulse device (rather than as a DC device).  Each pulse causes the stepper motor to advance by increments, which motion is imparted through the associated gearing (within gear box 84) to pinion gear 76.  As indicated in FIGURE 13, a mechanical ratchet 138 is provided between stepper motor 82 and the gearing within gear box 84 to assure one-way drive.  Rotation of pinion gear 76 causes longitudinal movement of beam 63 (or beam 91 if a non-disposable unit) to thus move the syringe piston a precise distance to thereby deliver the exact amount of medicament prescribed.

A stepper motor is utilized in this invention for reasons of safety in the case of an accidental conductive path forming between the power supply of the device and the motor, which with DC motors causes uncontrolled motion.  A stepper motor, on the other hand, requires clearly defined pulses in order to operate.  Stepper motors were not heretofore utilized for battery-operated devices, however, since one disadvantage of such motors ws high power consumption.

By this invention, the safety benefits of the stepper motor have been achieved by utilization of power efficient

-16-

means for driving the stepper motor which has allowed use of the stepper motor without requiring high power consumption and thus has permitted use of the stepper motor with a battery operated device.

The power efficient means for operating the stepper motor is illustrated by the pulse timing illustrations of FIGURE 16. The pulse timing illustrated in FIGURES 16(A) and 16(B) are manufacturer drive pulse recommendations (FIGURE 16(A)), and pulses as utilized in connection with a stepper motor as reported in NASA Technical Briefs, Volume 8, No. 4, MFS-25119, Summer 1984 (FIGURE 16(B)). The conventional step drive shown in FIGURE 16(A) is powered on between steps and energy efficiency is low, while the drive is partially powered on between steps in the drive shown in FIGURE 16(B) with energy efficiency being moderate.

FIGURE 16(C) illustrates pulses in a ballistic drive utilizable in conjunction with a unidirectional rachet presenting known inertia mounted on the motor shaft and including a pawl for establishing the direction of rotation. FIGURE 16(D) illustrates drive pulses that consume less energy than those previously shown in FIGURE 16 and which embody a dynamic braking effect. FIGURE 16(E) illustrates drive pulses allowing precise design of energy consumption and force development by the motor by means of duty cycle programming of the pulses. All of the drive pulses shown in FIGURES 16 C, D, and E can be utilized in this invention, to provide high energy efficiency, with the drive pulses as shown in FIGURE 16(E) being now preferred.

The pulse parameters are chosen in view of the desired inertia presented to the motor and in view of the maximum force to be allowed for any particular application.

As also indicated in FIGURE 13, an internal memory check is provided by memory check unit 140, and a clock check is provided by clock check unit 142, which unit may be connected to clocks 144 and 146. In addition, batteries 148 and 149 are connected to power the device through a diode type network 150, and a mini-battery 151 is provided for back-up. A control strip 153 is also provided for connection with the state machine.

Strip sensor 78 is positioned adjacent to side 71 of beam 63 to sense positioning of strips 70 thereon. As indicated in FIGURE 13, the output of sensor 78 is provided to state machine 122. In addition, rotation of the gearing within the gear box 84 (which gearing is connected with pinion gear 76 to cause movement of the rack and syringe piston) is sensed by rotation sensor 154 (which sensing can be accomplished, for example, by placing holes in an associated gear 155 and providing a light generator at one side and a sensor 154 at the opposite side). The output from sensor 154 is also provided to state machine 122. By thus sensing longitudinal movement of the beam and gear rotation, positioning control is enhanced and verified.

An alternate embodiment 158 is shown in FIGURE 14 for driving the syringe piston . As shown, a hinged rack 160 is provided to apply drive to the piston 47. To accomplish this type of drive, a plurality of rack sections 162 are provided each of which is connected with its adjacent section through a hinge pin 164. One end 166 of the thus formed hinge assembly is connected with the piston 47, andthe teeth 168 on each section 162 are sequentially brought into engagement with gear 170, which gear is in engagement with worm gear 172 rotated by stepper motor 82.

In addition, while an external plug 174 is indicated, internal battery operation can be utilized in the same manner as described herein above with respect to the other embodiments described herein.

Operation is essentially the same as with the previously described embodiments, except that the driving mechanism can be adjacent to the syringe and less longitudinal space is required due to hinging of the rack.

Another drive embodiment 178 is shown in FIGURE 15. As indicated, precise rotation of shaft 180 is provided by a one-way (clockwise as indicated) roller device unit 182. In this embodiment, rotation of the shaft is accomplished using balls 184 engaging the shaft. Each ball 184 is within a channel 186 having a slot 188 therein. Slot 188 is a longitudinally extending slot that is sufficiently wide to allow the ball to partly protrude there through so that the ball engages the shaft. In addition, each ball 184 is biased toward the end of the channel by a spring 190.

Clockwise rotation of the shift is permitted since the ball is being rotated by such movement in a direction away from the restraining wall of the channel. Counterclockwise rotation of the shaft is precluded, however, since attempted rotation of the shaft in this direction forces the ball into the restraining wall of the channel. Through use of this type of drive, rotation of shaft 180 controls movement of the syringe piston and no ratchet mechanism is required.

In operation of the disposable device of this invention, the disposable unit, with a predetermined amount of medicament in each syringe to be utilized, is loaded into the drive device while the lid of the casing is in an open position, by positioning the syringes in the syringe indentations at the top of the base portion as the mounting bracket is snapped into place at the front of the base portion. When so loaded, the teeth on the rack are brought into engagement with the pinion. The casing lid is then closed and locked by the mounting bracket snapping into the lid of the casing at closing. After insertion of a pre-programmed memory unit into the casing (through a closeable aperture (not shown) in the bottom of the casing), the unit is ready for use in dispensing medicament to a patient. In use, each syringe is connected with the patient in conventional fashion and medicament is thereafter delivered to the patient under the control of the pre-programmed memory unit.

When the medicament in the syringe (or syringes) is depleted, the disposable unit is removed from the drive device and discarded. If needed, a new unit can then be placed in the drive device for continued operation. Such a new unit can contain the same or different medicaments as determined to be needed by the patient's history.

The non-disposable unit operates in essentially the same manner as does the disposable unit, except that only the syringes and pistons are introduced into the unit (either individually or as a pack) with each syringe being positioned on the base unit and each piston being then connected with a separate rack. After use, each piston is disconnected from the rack, and the syringe then taken from the base unit and discarded.

-21-

As can be appreciated from the foregoing, this invention provides an improved device and method for controlling delivery of fluid from syringes.

-22-

WHAT IS CLAIMED IS:

1. A device for controlling delivery of fluid from a syringe having a piston the movement of which in a predetermined direction causes said fluid to be expelled from said syringe, said device comprising: a stepper motor having a rotatable motor shaft; pulse drive means connected with said stepper motor to provide pulses thereto for causing rotation of said motor shaft by increments; a housing having a receiving portion for receiving said syringe thereon; and motion translation means mounted on said housing and connected with said motor shaft and said piston for causing movement of said piston in said predetermined direction in response to said incremental rotation of said motor shaft.

2. The device as claimed in claim 1 wherein said motion translation means includes either a rack and pinion, a hinged rack and pinion or a one-way ball drive.

3. The device as claimed in claim 2 wherein said motion translation means includes said rack and pinion and said pinion is connected with said motor shaft through a gearing arrangement.

4. The device as claimed in claim 3 wherein said rack is a beam having teeth disposed therein and said pinion meshes with the teeth of said beam.

-23-

5. The device as claimed in any one of claims 3-4, wherein said device includes sensing means for separately sensing movement of said rack and pinion for determining that fluid has been discharged from said syringe.

6. The device as claimed in claim 5 wherein said rack has distinguishable strips thereon, wherein said pinion is connected with said drive means through gear means having apertures therein, and wherein said sensing means includes a first sensor responsive to said distinguishable strips on said rack, and second sensor responsive to movement of said apertures in said gear means.

7. The device as claimed in any one of claims 3-6 wherein said device includes a retaining clip having said rack mounted thereon, said clip being engageable with said housing for mounting of said syringe and rack at a rack portion of said housing, and with said retaining clip, syringe, and beam being assembled as a disposable unit.

8. The device as claimed in any one of claims 3-7 wherein said device controls delivery of fluid from a plurality of syringes, wherein said receiving portion of said housing receives said plurality of syringes thereat, wherein a separate rack is provided for each said syringe, and wherein said drive means includes a separate pinion gear for each of said beams.

-24-

9. The device as claimed in claim 8 wherein said racks are mounted on said base unit, and wherein each of said racks has connector means at one end for connecting each said rack to a different one of said syringe pistons.

10. The device as claimed in claim 9 wherein each of said connector means includes snap means engageable with cooperable means on said one end of said racks for releasably maintaining said connector means in engagement with its associated rack.

11. The device as claimed in any one of claims 8-10 wherein a retainng clip includes locking means engageable with end of said syringes for releasably locking said syringes on said retaining clip.

12. The device as claimed in claim 11 wherein said locking means includes arcuate sections matable with arcuate sections on said syringes.

13. The device as claimed in any one of the preceding claims wherein said device includes control means having a pre-programmed memory unit for controlling operation of said motion translation means to effect discharge of fluid from said syringe in predetermined amounts over predetermined periods of time.

-25-

14. A method for controlling delivery of fluid from a syringe having a piston the movement of which in one direction causes discharge of fluid from said syringe, said method comprising:

positioning said syringe on a retaining rack;

attaching said rack to said piston to said syringe to effect movement of said piston responsive to movement of said rack; and

providing a drive unit with a pinion gear at said rack for engaging the teeth of said rack to effect controlled movement of said piston.

15. The method of claim 14, wherein said drive unit includes a stepping motor for incrementally driving said pinion gear and further including the step of sensing the positioning of said rack and said pinion gear in order to verify.

0204977

Fig_1

Fig_2

Fig_3

Fig_4

Fig‒5

0204977

Fig_6

Fig_7

*Fig_8*

Fig_9

Fig_10

Fig_11

Fig_10A

0204977

6/9

*Fig_ 12*

SYRINGE PACK *120*

DRIVE MODULE *121*

VISUAL INDICATOR *29*

REMOTE DEMAND OPTION

*122*

BATTERIES

PROGRAMMED MEMORY

*126*

*130*

BEEPER

*42* ~ ENABLE    START    STOP

*124*  *124*

LED   LED

*128*

HISTORY

*132*

*39*    *40*

*178*

*186*
*180*
*184*
*190*
*182*
*184*
*188*
*188*
*190*
*186*
*188.*
*184*
*190*
*186*

*Fig_15*

Fig.16A

CONVENTIONAL DRIVE

Fig.16B

STEP  STEP  STEP  STEP  STEP

ALTERNATE KOWN DRIVE

Fig.16C

BALLISTIC DRIVE

Fig.16D

BRAKE  BRAKE  BRAKE  BRAKE

PARTIAL STEP DRIVE

Fig.16E

DRIVE VOLTAGE

IMI PARTIAL STEP SWITCHING DRIVE

TIME →

Fig_ 13

Fig_14

0204977

# EUROPEAN SEARCH REPORT

European Patent Office

## DOCUMENTS CONSIDERED TO BE RELEVANT

| Category | Citation of document with indication, where appropriate, of relevant passages | Relevant to claim | CLASSIFICATION OF THE APPLICATION (Int. Cl 4) |
|---|---|---|---|
| X | US-A-4 342 311 (G. WHITNEY et al.) <br> * Column 16, lines 33-50; figures 2,12,13,16 * <br> --- | 1,13 | A 61 M    5/14 |
| X | US-A-4 417 889 (CHOI) <br> * Figures 2,3; column 3, lines 9-16 * | 1,13 | |
| A | --- | 5,6 | |
| A | US-A-3 858 581 (D. KAMEN) <br> * Column 5, lines 48-58; figure 3 * <br> --- | 1 | |
| A | EP-A-0 092 712 (INTERMEDICAT) <br> * Figures 1-6; page 5, lines 12-14 * <br> --- | 1-4,10 ,11 | TECHNICAL FIELDS SEARCHED (Int. Cl.4) <br><br> A 61 M |
| A | US-A-4 255 096 (G.M. COKER et al.) <br> * Figure 1; column 1, lines 36-38; column 2, lines 33-38 * <br> --- | 8-12 | |
| A | GB-A-2 109 242 (FRESENIUS) <br><br> * Figures 1-3; page 2, lines 21-24,51-54,62-69 * <br> --- | 1-7,9-12 | |

The present search report has been drawn up for all claims

| Place of search | Date of completion of the search | Examiner |
|---|---|---|
| THE HAGUE | 04-09-1986 | RAKOWICZ,J.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO Form 1503 03 82